(19) Europäisches Patentamt
European Patent Office
Office européen des brevets

(11) Numéro de publication : **0 445 023 B1**

(12) **FASCICULE DE BREVET EUROPEEN**

(45) Date de publication du fascicule du brevet : **10.02.93 Bulletin 93/06**

(51) Int. Cl.$^5$ : **A61K 47/18**

(21) Numéro de dépôt : **91400519.4**

(22) Date de dépôt : **26.02.91**

(54) **Excipient pour principe actif thérapeutique à usage externe et préparation antiseptique comprenant cet excipient.**

(30) Priorité : **26.02.90 FR 9002355**

(43) Date de publication de la demande : **04.09.91 Bulletin 91/36**

(45) Mention de la délivrance du brevet : **10.02.93 Bulletin 93/06**

(84) Etats contractants désignés : **BE CH DE DK ES GB IT LI LU NL SE**

(56) Documents cités :
**EP-A- 0 303 713**
**US-A- 4 525 200**

(73) Titulaire : **INNOTHERA**
**10, avenue Paul Vaillant Couturier**
**F-94110 Arcueil (FR)**

(72) Inventeur : **Lequien, Gérard**
**52, route de Corcelles**
**F-21000 Dijon (FR)**
Inventeur : **Lallier, Michel, Léandre**
**55, avenue Jean-Jaurès**
**F-94250 Gentilly (FR)**

(74) Mandataire : **Kedinger, Jean-Paul et al**
**c/o Cabinet Malemont 42, avenue du Président Wilson**
**F-75116 Paris (FR)**

**EP 0 445 023 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet un excipient pour un principe actif thérapeutique à usage externe tel qu'un antiseptique du type composé organochloré de la classe des carbanilides ; elle concerne également une préparation antiseptique comprenant cet excipient.

L'une des difficultés rencontrées lors de la mise au point d'une préparation antiseptique à usage externe, réside dans le choix de l'excipient devant être associé au principe actif antiseptique. En effet, cet excipient doit répondre à au moins trois conditions essentielles. Il doit être physiologiquement toléré par les tissus sur lesquels il est destiné à être appliqué ; il doit ensuite être capable de solubiliser le principe actif dans une mesure suffisante pour que la préparation résultante ait l'activité antiseptique recherchée et notamment une activité antiseptique telle que définie dans la Pharmacopée Française Xème Edition au chapitre consacré à la monographie des antiseptiques ; il doit enfin assurer une bonne stabilité de la préparation, notamment lorsque celle-ci est soumise à une dilution.

Les composés organochlorés de la classe des carbanilides, et en particulier le trichloro-3,4,4′ carbanilide, font preuve d'une activité bactériostatique, bactéricide et fongicide intéressante et trouvent donc leur utilisation en tant que principes actifs antiseptiques. Toutefois, ces composés sont insolubles dans l'eau et très peu solubles dans les solvants habituellement utilisés pour des usages externes, ce qui en limite l'intérêt pratique. On a donc cherché, dans le passé, à mettre au point des excipients pour lesdits composés organochlorés ; il s'avère néanmoins que les préparations obtenues avec ces excipients ne présentent pas une activité antiseptique satisfaisant aux critères d'activité définis dans la Pharmacopée Française susmentionnée.

Le but de la présente invention est par conséquent de proposer un nouvel excipient pour principe actif thérapeutique à usage externe tel qu'un composé organochloré de la classe des carbanilides, répondant aux trois conditions rappelées ci-dessus. Conformément à l'invention, cet excipient se caractérisé en ce qu'il comprend :

- un composé de formule :

$$R_1 - {}^{\oplus}NR_2R_2R_3 \qquad (Ia)$$

où $R_1$ = alkyle linéaire en $C_{12}$, $R_2 = (CH_2CH_2O)_2H$ et $R_3 = CH_2COO^-$ , et

- un composé de formule :

$$R_1 - \overset{\overset{O}{\uparrow}}{N}(CH_3)_2 \qquad (IIa)$$

où $R_1$ = alkyle linéaire en $C_{12}$.

Du point de vue de la tolérance, on préfère selon l'invention, que l'excipient comprenne en outre au moins un composé choisi parmi ceux de formule :

$$R_4 - {}^{\oplus}NR_2R_2R_3 \qquad (Ib)$$

où $R_4$ = alkyle linéaire en $C_8$, $C_{10}$, $C_{14}$, $C_{16}$ ou $C_{18}$ et $R_2$ et $R_3$ ont la même signification que dans la formule (Ia) et au moins un composé choisi parmi ceux de formule :

$$R_5 - \overset{\overset{O}{\uparrow}}{N}(CH_3)_2 \qquad (IIb)$$

où $R_5$ = alkyle linéaire en $C_{14}$ ou $C_{16}$.

Les composés (Ia) et (Ib) sont des agents tensio-actifs amphotères solubilisants. Tout comme les composés (IIa) et (IIb), ils conjuguent une très bonne solubilité dans l'eau, avec un pouvoir bactéricide propre, ce qui présente un intérêt tout particulier lorsque ledit excipient est destiné à la formulation d'une préparation comprenant un principe actif antiseptique puisqu'au sein de cette préparation il y aura cumul du pouvoir bactéricide de ce principe actif et de celui de l'excipient.

Avantageusement, les composés (Ib) ci-dessus où $R_4$ désigne un groupe alkyle linéaire en $C_8$ et $C_{10}$ représentent au plus 10 % et les composés (Ib) où $R_4$ désigne un groupe alkyle linéaire en $C_{14}$, $C_{16}$ ou $C_{18}$ représentent respectivement 17-24 %, 6-12 % et 5-10 %, ces pourcentages étant donnés par rapport à la somme des poids du composé de formule (Ia) et des composés de formule (Ib).

Quant au composé (IIb) où $R_5$ = alkyle linéaire en $C_{14}$ et au composé (IIb) où $R_5$ = alkyle linéaire en $C_{16}$,

ils sont avantageusement mis en oeuvre respectivement à raison de 27-31 % et 2,5-3,5 % de la somme des poids du composé de formule (IIa) et des composés de formule (IIb).

Par ailleurs, on peut notamment choisir les quantités des composés (Ia), (IIa), (Ib) et (IIb) de telle sorte que les rapports en poids composé (IIa)/composé (Ia) et composé (IIa) + composé(s) (IIb)/composé (Ia) + composé(s) (Ib) soient compris chacun dans l'intervalle de 0,2 à 0,8.

Pour améliorer encore la tolérance de l'excipient, ce dernier peut de plus comprendre :

- un dérivé de polypeptide (IIIa) obtenu par condensation en milieu acide, notamment l'acide chlorhydrique, de l'amide de formule :

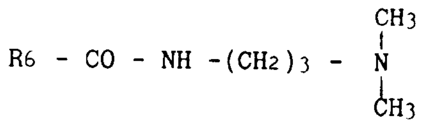

(IVa)

où $R_6$ = alkyle linéaire en $C_{11}$, avec un polypeptide possédant une masse moléculaire moyenne de 300 à 2000, et

- de préférence au moins un dérivé de polypeptide (IIIb) choisi parmi ceux obtenus par condensation, en milieu acide, notamment l'acide chlorhydrique, des amides de formule :

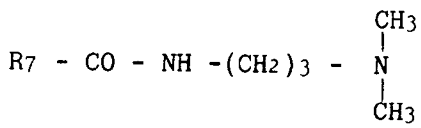

(IVb)

où $R_7$ désigne un groupe alkyle linéaire en $C_7$, $C_9$, $C_{13}$, $C_{15}$ ou $C_{17}$, avec un polypeptide possédant une masse moléculaire moyenne de 300 à 2000.

Selon un mode de réalisation de l'excipient, les dérivés (IIIb) obtenus par mise en oeuvre des amides de formule (IVb) où $R_7$ désigne un groupe alkyle linéaire en $C_7$ et $C_9$ représentent au plus 10 % de la somme des poids du dérivé (IIIa) et des dérivés (IIIb) et les dérivés (IIIb) obtenus par mise en oeuvre des amides de formule (IVb) où $R_7$ désigne un groupe alkyle linéaire en $C_{13}$, $C_{15}$ et $C_{17}$ représentant respectivement 17-24 %, 6-12 % et 5-10 % de cette même somme.

Le polypeptide entrant dans la formation des dérivés (IIIa) et (IIIb) est par exemple constitué par un polypeptide collagénique obtenu par hydrolyse de la gélatine.

D'autre part, il est souhaitable que les rapports en poids dérivé (IIIa)/composé (Ia), dérivé (IIIa)/composé (Ia) + composé(s) (Ib), dérivé (IIIa) + dérivé(s) (IIIb)/composé (Ia) et dérivé (IIIa) + dérivé(s) (IIIb)/composé (Ia) + composé(s) (Ib) soient chacun inférieurs ou égaux à 0,4.

L'excipient selon l'invention peut en outre comprendre de l'eau dans laquelle les composés et dérivés (Ia), (Ib), (IIa), (IIb), (IIIa) et (IIIb) sont aisément miscibles.

Lorsque cet excipient est destiné à entrer dans la composition d'une préparation antiseptique susceptible d'être diluée dans de l'eau de dureté variable avant utilisation, il est avantageux, en vue d'augmenter la stabilité de la solution diluée résultante, que ledit excipient contienne un agent complexant des ions alcalino-terreux, tel que l'EDTA, les sels sodiques de l'EDTA et leurs mélanges, les rapports en poids agent complexant/composé (Ia) et agent complexant/composé (Ia) + composé(s) (Ib) étant chacun de préférence inférieurs ou égaux à 0,1.

Enfin, l'adjonction d'un agent d'ajustement du pH à 4-7, tel que l'acide citrique, l'acide lactique et/ou le citrate trisodique, peut être souhaitable pour conférer à l'excipient un pH compatible avec les tissus avec lesquels il est destiné à être mis en contact.

Des excipients préférés selon l'invention, répondent à la composition ci-après où les valeurs de pourcentage données entre parenthèses correspondent à des excipients tout particulièrement préférés :

- composé (Ia) + composés (Ib) ...... 7,5 - 15 (9,5-15)% en poids
- composé (IIa) + composés (IIb) ..... 3 - 6 (4-5) % en poids
- dérivé (IIIa) + dérivés (IIIb) ..... 0 - 3 (0,15-1,5)% en poids
- agent complexant ................... 0 - 0,5 % en poids
- agent d'ajustement du pH à 4-7
- eau déminéralisée .................. q.s.q. 100 % en poids

Les composés (Ia) et (Ib) mis en oeuvre selon l'invention peuvent être obtenus par condensation, en milieu aqueux à 70-80° C, du sel de sodium de l'acide monochloracétique sur une amine tertiaire de formule $R_1R_2R_2N$ ou $R_4R_2R_2N$ où $R_1$, $R_2$ et $R_4$ ont la même signification que dans les formules (Ia) et (Ib). Une telle réaction de condensation pourra par exemple être effectuée selon la méthode décrite dans Amphoteric surfactant - Surfactant Sciences Series Vol. 12 - DEKKER page 75. Les composés (Ia) et (Ib) sont des produits commercialisés par la société française SEPPIC, sous la marque AMONYL® 640NL.

Les composés (IIa) et (IIb) peuvent pour leur part être préparés par oxydation par le peroxyde d'hydrogène en milieu aqueux, notamment à 70° C, d'une amine tertiaire de formule

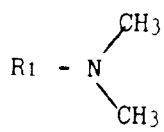 ou 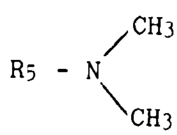

où $R_1$ et $R_5$ ont la même signification que dans les formules (IIa) et (IIb). Les composés (IIa) et (IIb) sont des produits commercialisés par la société AKZO, sous la marque AROMOX®, notamment AROMOX® DMMCD-W.

Quant aux composés (IVa) et (IVb), ils peuvent être obtenus par action de l'acide gras de formule $R_6$-COOH ou $R_7$-COOH sur la diméthylamino propylamine en milieu alcalin. D'autre part, le polypeptide à condenser avec ces composés (IVa) et (IVb) peut être préparé par hydrolyse, par exemple selon la technique décrite dans US-A-3 738 913, de protéines du type gélatine.

Il est à noter par ailleurs que les dérivés (IIIa) et (IIIb) sont des produits commercialisés sous la marque de fabrique MONTEINE® LCQ par la société française SEPPIC (Société d'Exploitation de Produits pour les Industries Chimiques).

L'excipient ainsi décrit présente un intérêt tout particulier pour la formulation d'une préparation antiseptique dont le principe actif est un composé organochloré insoluble dans l'eau, de la classe des carbanilides, notamment le trichloro-3,4,4′ carbanilide.

Selon un mode de réalisation préféré, cette préparation antiseptique comprend les constituants suivants :

- trichloro-3,4,4' carbanilide ................. 1,0 % en poids
- composé (Ia) + composés (Ib) ................. 11,5 % en poids
- composé (IIa) + composés (IIb) ............... 4,5 % en poids
- dérivé (IIIa) + dérivés (IIIb) ............... 0,9 % en poids
- agent complexant ............................. 0,3 % en poids
- agent d'ajustement du pH 4-7
- eau déminéralisée ............................ q.s.p. 100 % en poids

Diverses études de stabilité, de tolérance et d'activité ont été effectuées sur la préparation antiseptique selon l'invention.

En ce qui concerne la stabilité, la formation d'aucun trouble ou précipitation de la préparation n'a pu être constatée après 6 mois ; en particulier, une dilution de 30 ml de ladite préparation dans 250 ml d'eau a conduit à une solution diluée stable pendant au moins 6 mois.

Par ailleurs, les études de tolérance oculaire et primaire cutanée chez l'animal ont montré que la solution diluée susmentionnée n'est que très faiblement irritante sur l'oeil et non irritante sur la peau. L'application cutanée itérative dans les conditions normales d'emploi pendant 4 semaines chez l'animal n'entraîne pas d'irritation particulière. De plus, des études ex vivo effectuées sur la peau épilée ou rasée de rats, n'ont pas permis de déceler un passage transcutané du principe actif.

D'autres part, la détermination de l'activité bactéricide et fongicide de la préparation antiseptique a été menée vis-à-vis des cinq souches de référence suivantes : Pseudomonas aeruginosa C.I.P. A.22, Escherichia coli C.I.P. 54.127, Staphylococcus aureus C.I.P. 53.154, Enterococcus faecium C.I.P. 58.55 et candida albicans C.I.P. 1180.79.

L'étude a porté sur la préparation suivante :

- trichloro-3,4,4' carbanilide .................. 1,0 % en poids
- composé (Ia) + composés (Ib) .................. 11,5 % en poids
- composé (IIa) + composés (IIb) ................ 4,5 % en poids
- dérivé (IIIa) + dérivés (IIIb) ................ 0,9 % en poids
- sel sodique d'EDTA ........................... 0,3 % en poids
- agent d'ajustement du pH (acide lactique) à pH environ 5
- eau déminéralisée ........................... q.s.p. 100 % en poids

dans les conditions expérimentales ci-après :

- dilution préalable à raison de 30 ml dans 250 ml d'eau purifiée,
- temps de contact : 5 minutes
- méthode par filtration sur membranes, conforme au protocole défini dans l'arrêté du 20.3.87 portant additif No. 5 à la Pharmacopée Française (Xème Edition).

Diverses concentrations de la préparation antiseptique selon l'invention ont été étudiées et les valeurs expérimentales de deux dénombrements, de la moyenne calculée et du pH sont rassemblées dans le tableau 1 ci-après :

TABLEAU 1

| SOUCHES, collection d'origine et numero dans la collection | | Numérations | | Concentrations essayées du produit au contact avec les microorganismes (% V/V) | | |
|---|---|---|---|---|---|---|
| | | initiale | terminale | 90 | 50 | 10 |
| Pseudomonas aeruginosa C.I.P. A22 | d | 185/193 | 169/211 | 0/0 | 0/0 | +/+ |
| | m | 189 | 190 | 0 | 0 | + |
| | pH | | | 4,9 | 5,1 | 6,1 |
| Escherichia coli C.I.P. 54.127 | d | 98/113 | 87/105 | 0/0 | 0/0 | 0/0 |
| | m | 106 | 96 | 0 | 0 | 0 |
| | pH | | | 4,8 | 5,1 | 6,1 |
| Staphylococcus aureus C.I.P. 53.154 | d | 96/102 | 92/91 | 0/0 | 0/0 | 17/16 |
| | m | 99 | 92 | 0 | 0 | 17 |
| | pH | | | 4,9 | 5,2 | 6,1 |
| Enterococcus faecium C.I.P. 58.55 | d | 108/115 | 134/164 | 1/0 | 1/1 | 3/1 |
| | m | 112 | 149 | 0 | 1 | 2 |
| | pH | | | 4,9 | 5,2 | 6,2 |
| Candida albicans C.I.P. 1180.79 | d | 158/167 | 147/170 | 5/8 | 38/52 | 149/172 |
| | m | 163 | 159 | 7 | 45 | 161 |
| | pH | | | 4,9 | 5,1 | 5,5 |

d : dénombrement (1er/2ème essai)
m : moyenne
\+ : plus de 200 colonies

Le tableau 1 ci-dessus montre qu'une réduction d'au moins 5 log (d'au moins $10^5$ fois) de la population en microorganismes est obtenue avec la composition testée aux concentrations (V/V) de :

- 90 et 50 % pour Pseudomonas aeruginosa C.I.P. A22
- 90, 50 et 10 % pour Escherichia coli C.I.P. 54.127
- 90, 50 et 10 % pour Staphylococcus aureus C.I.P.. 53.154
- 90, 50 et 10 % pour Enterococcus faecium C.I.P. 58.55
- 90 et 50 % pour Candida albicans C.I.P. 1180.79

La détermination de l'activité bactéricide et fongicide a été répétée en présence des substances interférentes suivantes :

- eau dure (titre final dans l'essai : 60° français ou 12 méq.) ;
- mélange d'albumine (1 % M/V) et d'extrait de levure (1 % M/V)

Le Tableau 2 ci-après rassemble les résultats comparatifs des plus faibles concentrations (V/V) de la

composition selon l'invention pour lesquelles une réduction de 5 log de la population en microorganismes a pu être obtenue.

TABLEAU 2

| | Substance(s) interférentes(s) | | |
|---|---|---|---|
| | Sans | Eau dure | Protéines |
| <u>Pseudomonas aeruginosa</u> C.I.P. A22 | 50 % | 50 % | 50 % |
| <u>Escherichia coli</u> C.I.P. 54.127 | 10 % | 10 % | 10 % |
| <u>Staphylococcus aureus</u> C.I.P. 53.154 | 10 % | 10 % | 50 % |
| <u>Enterococcus faecium</u> C.I.P. 58.55 | 10 % | 10 % | 10 % |
| <u>Candida albicans</u> C.I.P. 1180.79 | 50 % | 50 % | 10 % |

Les résultats qui précèdent démontrent le pouvoir antiseptique élevé de la préparation selon l'invention, qui en fait un moyen de choix pour les traitements antiseptiques dans les domaines les plus divers et notamment en dermatologie, cosmétologie, gynécologie et petite chirurgie, l'intérêt de cette composition étant encore accru par le bon pouvoir détergent dont sont à l'origine les propriétés moussantes des composés (Ia), (Ib), (IIa), et (IIb) entrant dans la constitution de l'excipient.

On ajoutera encore que la préparation antiseptique selon l'invention peut être utilisée pure ou diluée dans l'eau, notamment à 10-15 % (V/V), et de préférence à 12 % (V/V). En outre, la meilleure tolérance physiologique étant obtenue avec une préparation à pH 5-6,5 et la meilleure efficacité antiseptique du principe actif étant obtenue à 4,5-6, on ajustera avantageusement le pH de cette préparation à 5-6.

## Revendications

1. Excipient pour principe actif thérapeutique à usage externe, caractérisé en ce qu'il comprend :
   - un composé de formule :

$$R_1 - {}^{\oplus}NR_2R_2R_3 \qquad (Ia)$$

   où $R_1$ = alkyle linéaire en $C_{12}$, $R_2$ = $(CH_2CH_2O)_2H$ et $R_3$ = $CH_2COO^-$, et
   - un composé de formule :

$$R_1 - \overset{\overset{O}{\uparrow}}{N}(CH_3)_2 \qquad (IIa)$$

   où $R_1$ = alkyle linéaire en $C_{12}$.

**2.** Excipient selon la revendication 1, caractérisé en ce qu'il comprend en outre au moins un composé choisi parmi ceux de formule :

$$R_4 - {}^{\oplus}NR_2R_2R_3 \qquad (Ib)$$

où $R_4$ = alkyle linéaire en $C_8$, $C_{10}$, $C_{14}$, $C_{16}$ ou $C_{18}$ et $R_2$ et $R_3$ ont la même signification que dans la formule (Ia).

**3.** Excipient selon la revendication 2, caractérisé en ce que les composés de formule (Ib) où $R_4$ désigne un groupe alkyle linéaire en $C_8$ et $C_{10}$ représentent au plus 10 % et les composés (Ib) où $R_4$ désigne un groupe alkykle linéaire $C_{14}$, $C_{16}$ ou $C_{18}$ représentant respectivement 17-24 %, 6-12 % et 5-10 %, ces pourcentages étant donnés par rapport à la somme des poids du composé de formule (Ia) et des composés de formule (Ib).

**4.** Excipient selon l'une des revendications 1 à 3, caractérisé en ce qu'il comprend en outre au moins un composé choisi parmi ceux de formule :

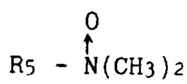

(IIb)

où $R_5$ = alkyle linéaire en $C_{14}$ ou $C_{16}$.

**5.** Excipient selon la revendication 4, caractérisé en ce que le composé de formule (IIb) où $R_5$ = alkyle linéaire en $C_{14}$ et le composé de formule (IIb) où $R_5$ = alkyle linéaire en $C_{16}$ représentent respectivement 27-31 % et 2,5-3,5 % de la somme des poids du composé de formule (IIa) et des composés de formule (IIb).

**6.** Excipient selon l'une des revendications précédentes, caractérisé en ce que les rapports en poids composé (IIa)/composé (Ia) et composé (IIa) + composé(s) (IIb)/composé (Ia) + composé(s) (Ib) sont compris chacun dans l'intervalle de 0,2 à 0,8.

**7.** Excipient selon l'une des revendications précédentes, caractérisé en ce qu'il comprend de plus un dérivé de polypeptide (IIIa) obtenu par condensation en milieu acide, de l'amide de formule :

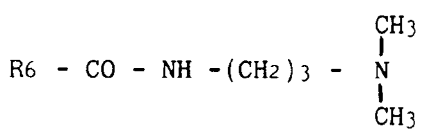

(IVa)

où $R_6$ = alkyle linéaire en $C_{11}$ avec un polypeptide possédant une masse moléculaire moyenne de 300 à 2000.

**8.** Excipient selon la revendication 7, caractérisé en ce qu'il comprend de plus au moins un dérivé de polypeptide (IIIb) choisi parmi ceux obtenus par condensation en milieu acide, des amides de formule :

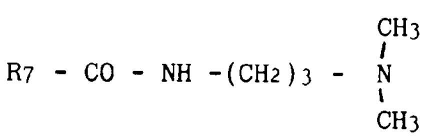

(IVb)

où $R_7$ = alkyle linéaire en $C_7$, $C_9$, $C_{13}$, $C_{15}$ ou $C_{17}$ avec un polypeptide possédant une masse moléculaire moyenne de 300 à 2000.

**9.** Excipient selon la revendication 8, caractérisé en ce que les dérivés (IIIb) obtenus par mise en oeuvre des amides de formule (IVb) pour lesquels $R_7$ désigne alkyle linéaire en $C_7$ et $C_9$ représentent au plus 10 % de la somme des poids du dérivé (IIIa) et des dérivés (IIIb) et en ce que les dérivés (IIIb) obtenus par

mise en oeuvre des amides de formule (IVb) pour lesquels $R_7$ désigne alkyle linéaire en $C_{13}$, $C_{15}$ et $C_{17}$ représentent respectivement 17-24 %, 6-12 % et 5-10 % de cette même somme.

**10.** Excipient selon la revendication 7, 8 ou 9, caractérisé en ce que le polypeptide est un hydrolysat de gélatine.

**11.** Excipient selon les revendications 7, 8, 9 ou 10, caractérisé en ce que les rapports en poids dérivé (IIIa)/composé (Ia), dérivé (IIIa)/composé (Ia) + composé(s) (Ib), dérivé (IIIa) + dérivé(s) (IIIb)/composé (Ia) et dérivé (IIIa) + dérivé(s) (IIIb)/composé (Ia) + composé(s) (Ib) sont chacun inférieurs ou égaux à 0,4.

**12.** Excipient selon l'une des revendications précédentes, caractérisé en ce qu'il comprend de l'eau.

**13.** Excipient selon l'une des revendications précédentes, caractérisé en ce qu'il comprend un agent complexant des ions alcalino-terreux.

**14.** Excipient selon la revendication 13, caractérisé en ce que l'agent complexant est au moins l'un choisi parmi l'EDTA, les sels sodiques de l'EDTA et leurs mélanges.

**15.** Excipient selon la revendication 13 ou 14, caractérisé en ce que les rapports en poids agent complexant/composé (Ia) et agent complexant/composé (Ia) + composé(s) (Ib) sont chacun inférieurs ou égaux à 0,1.

**16.** Excipient selon l'une des revendications 12 à 15, caractérisé en ce qu'il contient un agent d'ajustement du pH à 4-7.

**17.** Excipient selon l'une des revendications 12 à 16, caractérisé en ce qu'il comprend :

| | |
|---|---|
| - composé (Ia) + composés (Ib) .............. | 7,5 - 15 % en poids |
| - composé (IIa) + composés (IIb) ............ | 3 - 6 % en poids |
| - dérivé (IIIa) + dérivés (IIIb) ............ | 0 - 3 % en poids |
| - agent complexant .......................... | 0 - 0,5 % en poids |
| - agent d'ajustement du pH à 4-7 | |
| - eau déminéralisée ......................... | q.s.q. 100 % en poids |

**18.** Préparation antiseptique comprenant un composé organochloré insoluble dans l'eau, de la classe des carbanilides et un excipient, caractérisée en ce que l'excipient est constitué par celui objet de l'une quelconque des revendications 1 à 17.

**19.** Préparation antiseptique selon la revendication 18, caractérisée en ce que ledit composé organochloré est constitué par le trichloro-3,4,4′ carbanilide.

**20.** Préparation antiseptique selon la revendication 18 ou 19, caractérisée en ce qu'elle contient 0,5 à 2,5 % en poids dudit composé organochloré de la classe des carbanilides.

**21.** Préparation antiseptique selon la revendication 20, caractérisée en ce qu'elle comprend les constituants suivants :

| | |
|---|---|
| - trichloro-3,4,4' carbanilide ................. | 1,0 % en poids |
| - composé (Ia) + composés (Ib) ................. | 11,5 % en poids |
| - composé (IIa) + composés (IIb) ............... | 4,5 % en poids |
| - dérivé (IIIa) + dérivés (IIIb) ............... | 0,9 % en poids |
| - agent complexant ............................. | 0,3 % en poids |
| - agent d'ajustement du pH à 4-7 | |
| - eau déminéralisée ............................ | q.s.p. 100 % en poids |

## Patentansprüche

1. Träger für therapeutischen Wirkstoff zur äußeren Anwendung, dadurch gekennzeichnet, daß er
   - eine Verbindung der Formel

   $$R_1 - {}^{\oplus}NR_2R_2R_3 \qquad (Ia),$$

   wo $R_1$ = lineares Alkyl aus $C_{12}$, $R_2 = (CH_2CH_2O)_2H$ und $R_3 = CH_2COO^-$, und
   - eine Verbindung der Formel

   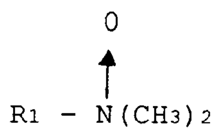

   (IIa),

   wo $R_1$ = lineares Alkyl aus $C_{12}$ enthält.

2. Träger nach Anspruch 1, dadurch gekennzeichnet, daß er außerdem von den Verbindungen der Formel

   $$R_4 - {}^{\oplus}NR_2R_2R_3 \qquad (Ib)$$

   mindestens eine enthält, wo $R_4$ = lineares Alkyl aus $C_8$, $C_{10}$, $C_{14}$, $C_{16}$ oder $C_{18}$ und wo $R_2$ und $R_3$ die gleiche Bedeutung wie in der Formel (Ia) haben.

3. Träger nach Anspruch 2, dadurch gekennzeichnet, daß die Verbindungen mit der Formel (Ib), wo $R_4$ eine lineare Alkylverbindung aus $C_8$ und $C_{10}$ bezeichnet, höchstens 10% darstellen und die Verbindungen (Ib), wo $R_4$ eine lineare Alkylverbindung aus $C_{14}$, $C_{16}$ oder $C_{18}$ bezeichnet, jeweils 17-24%, 6-12% und 5-10% darstellen, diese Prozentsätze werden im Verhältnis der Summe der Gewichte der Verbindungen der Formel (Ia) und der Verbindungen der Formel (Ib) angegeben.

4. Träger nach einem der Ansprüche 1 bis 3, dadurch gekennzeichnet, daß er außerdem von den Verbindungen der Formel

   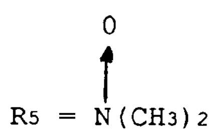

   (IIb)

   mindestens eine enthält, wo $R_5$ = lineares Alkyl aus $C_{14}$ oder $C_{16}$.

5. Träger nach Anspruch 4, dadurch gekennzeichnet, daß die Verbindung der Formel (IIb), wo $R_5$ = lineares Alkyl aus $C_{14}$ und die Verbindung der Formel (IIb), wo $R_5$ = lineares Alkyl aus $C_{16}$, jeweils 27-31% und 2,5-3,5% der Summe der Gewichte der Verbindung der Formel (IIa) und der Verbindungen der Formel (IIb) darstellen.

6. Träger nach einem der vorhergehenden Ansprüche, dadurch gekennzeichnet, daß die Gewichtsverhältnisse Verbindung (IIa) zu Verbindung (Ia) und Verbindung (IIa) + Verbindung(en) (IIb) zu Verbindung (Ia) + Verbindung(en) (Ib) jeweils im Bereich von 0,2 bis 0,8 liegen.

7. Träger nach einem der vorhergehenden Ansprüche, dadurch gekennzeichnet, daß er zusätzlich ein Polypeptidderivat (IIIa) enthält, das durch Kondensierung im sauren Medium des Amids der Formel

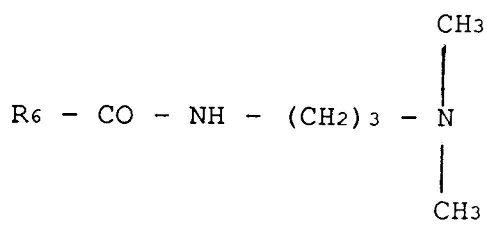

(IVa),

wo $R_6$ = lineares Alkyl aus $C_{11}$, mit einem Polypeptid mit einer durchschnittlichen Molekularmasse von 300 bis 2000 erhalten wird.

8. Träger nach Anspruch 7, dadurch gekennzeichnet, daß er außerdem wenigstens ein Polypeptidderivat (IIIb) enthält, das aus den durch Kondensierung im sauren Medium erhaltenen Amiden der Formel

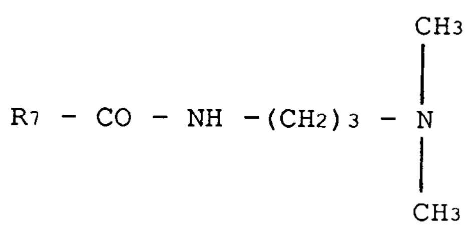

(IVb)

wo $R_7$ = lineares Alkyl aus $C_7$, $C_9$, $C_{13}$, $C_{15}$ oder $C_{17}$, mit einem Polypeptid mit einer durchschnittlichen Molekularmasse von 300 bis 2000 ausgewählt wird.

9. Träger nach Anspruch 8, dadurch gekennzeichnet, daß die Derivate (IIIb), die unter Anwendung der Amide der Formel (IVb) erhalten wurden, bei welchen $R_7$ lineare Alkyle aus $C_7$ und $C_9$ bezeichnet, höchstens 10% der Summe der Gewichte des Derivats (IIIa) und der Derivate (IIIb) darstellen, und daß die Derivate (IIIb), die unter Anwendung der Amide der Formel (IVb) erhalten wurden, bei welchen $R_7$ lineare Alkyle aus $C_{13}$, $C_{15}$ und $C_{17}$ bezeichnet, jeweils 17-24%, 6-12% und 5-10% dieser Summe darstellen.

10. Träger nach Anspruch 7, 8 oder 9, dadurch gekennzeichnet, daß der Polypeptid ein Gelatinehydrolysat ist.

11. Träger nach Anspruch 7, 8, 9 oder 10, dadurch gekennzeichnet, daß die Gewichtsverhältnisse Derivat (IIIa)/ Verbindung (Ia), Derivat (IIIa)/ Verbindung (Ia) + Verbindung(en) (Ib), Derivat (IIIa) + Derivat(e) (IIIb)/Verbindung (Ia) und Derivat (IIIa) + Derivat(e) (IIIb)/Verbindung (Ia) + Verbindung(en) (Ib) jeweils kleiner als oder gleich 0,4 sind.

12. Träger nach den Ansprüchen 1 bis 11, dadurch gekennzeichnet, daß er Wasser enthält.

13. Träger nach den Ansprüchen 1 bis 12, dadurch gekennzeichnet, daß er ein komplexbildendes Agens der erdalkalischen Ionen enthält.

14. Träger nach Anspruch 13, dadurch gekennzeichnet, daß das komplexbildende Agens mindestens ein aus der Ethylen-Diamin-Tetra-Essigsäure (EDTA), den Natriumsalzen der EDTA und deren Mischungen ausgewähltes ist.

15. Träger nach Anspruch 13 oder 14, dadurch gekennzeichnet, daß die Gewichtsverhältnisse komplexbildendes Agens/Verbindung (Ia) und komplexbildendes Agens/Verbindung (Ia) + Verbindung(en) (Ib) jeweils kleiner als oder gleich 0,1 sind.

16. Träger nach den Ansprüchen 12 bis 15, dadurch gekennzeichnet, daß er ein pH-stabilisierendes Agens zum pH-Wert 4-7 enthält.

17. Träger nach einem der Ansprüche 12 bis 16, dadurch gekennzeichnet, daß er

- Verbindung (Ia) + Verbindungen (Ib) 7,5 - 15% des Gewichtes
- Verbindung (IIa) + Verbindungen (IIb) 3 - 6% des Gewichtes
- Derivat (IIIa) + Derivate (IIIb) ...0 - 3% des Gewichtes
- komplexbildendes Agens ................0 -0,5% des Gewichtes
- pH-stabilisierendes Agens zum pH-Wert 4-7
- entmineralisiertes Wasser ........bis zu 100% des Gewichtes

enthält.

18. Antiseptikum mit einer wasserunlöslichen chlororganischen Verbindung aus der Gruppe der Carbanilide und einem Träger, dadurch gekennzeichnet, daß der Träger aus einem der Träger nach den Ansprüchen 1 bis 17 besteht.

19. Antiseptikum nach Anspruch 18, dadurch gekennzeichnet, daß die chlororganische Verbindung aus dem Trichloro-3,4,4'-Carbanilid besteht.

20. Antiseptikum nach Anspruch 18 oder 19, dadurch gekennzeichnet, daß es zu 0,5 bis 2,5% des Gewichtes die chlororganische Verbindung aus der Gruppe der Carbanilide enthält.

21. Antiseptikum nach Anspruch 20, dadurch gekennzeichnet, daß es folgende Bestandteile enthält:

- Trichloro-3,4,4'-Carbanilid.............1,0% des Gewichtes
- Verbindung (Ia) + Verbindungen (Ib)....11,5% des Gewichtes
- Verbindung (IIa)+ Verbindungen (IIb)....4,5% des Gewichtes
- Derivat (IIIa) + Derivate (IIIb)........0,9% des Gewichtes
- komplexbildendes Agens .................0,3% des Gewichtes
- pH-stabilisierendes Agens zum pH-Wert 4-7
- entmineralisiertes Wasser ......bis zu 100% des Gewichtes

## Claims

1. Excipient for therapeutic active principle for external application, characterized in that it comprises :
   - a compound having the formula :

$$R_1 - {}^{\oplus}NR_2R_2R_3 \qquad (Ia)$$

   wherein :
   - $R_1 = C_{12}$ straight-chain alkyl ;
   - $R_2 = (CH_2CH_2O)_2H$ ; and
   - $R_3 = CH_2COO^-$ ; and
   - a compound having the formula :

$$R_1 - \overset{\displaystyle O \atop \uparrow}{N}(CH_3)_2 \qquad (IIa)$$

wherein $R_1 = C_{12}$ straight-chain alkyl.

2. Excipient according to Claim 1, characterized in that it further comprises at least one compound selected among those having the formula :

$$R_4 - {}^{\oplus}NR_2R_2R_3 \qquad (Ib)$$

wherein :
- $R_4 = C_8, C_{10}, C_{14}, C_{16}$ or $C_{18}$ straight-chain alkyl, and
- $R_2$ and $R_3$ have the same meaning as in the formula (Ia).

3. Excipient according to Claim 2, characterized in that the compounds having the formula (Ib) wherein $R_4$ stants for a $C_8$ and $C_{10}$ straight-chain alkyl group represent at most 10% and the compounds (Ib) wherein $R_4$ stands for a $C_{14}$, $C_{16}$ or $C_{18}$ straight-chain alkyl represent 17-24%, 6-12% and 5-10%, respectively, these percentages being given with respect to the sum of the weights of the compound of formula (Ia) and the compounds of formula (Ib).

4. Excipient according to anyone of Claims 1 to 3, chaaracterized in that it further comprises at least one compound selected among those having the formula :

$$R_5 - \overset{\displaystyle O \atop \uparrow}{N}(CH_3)_2 \qquad (IIb)$$

wherein $R_5 = C_{14}$ or $C_{16}$ straight-chain alkyl.

5. Excipient according to Claim 4, characterized in that the compound of formula (IIb) wherein $R_5 = C_{14}$ straight-chain alkyl and the compound of formula (IIb) wherein $R_5 = C_{16}$ straight-chain alkyl represent 27-31% and 2.5-3.5% respectively of the sum of the weights of the compound of formula (IIa) and the compounds of formula (IIb).

6. Excipient according to anyone of the previous Claims, characterized in that the compound (IIa)/compound (Ia) and compound (IIa) + compound(s) (IIb)/compound (Ia) + compound(s) (Ib) weight ratios are included in the interval of 0.2 - 0.8, respectively.

7. Excipient according to anyone of the previous Claims, characterized in that it further comprises a polypeptide derivative (IIIa) obtained by condensing in an acidic medium, the amide having the formula :

$$R_6 - CO - NH - (CH_2)_3 - \underset{\displaystyle CH_3}{\overset{\displaystyle CH_3}{N}} \qquad (IVa)$$

wherein $R_6 = C_{11}$ straight-chain alkyl,
with a polypeptide having an average molecular weight of 300 to 2,000.

8. Excipient according to Claim 7, characterized in that it further comprises at least one polypeptide dérivative (IIIb) selected among those obtained by condensing in an acidic medium, amides having the formula :

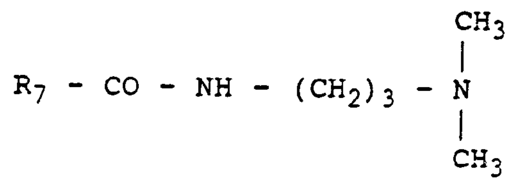 (IVb)

wherein $R_7 = C_7$, $C_9$, $C_{13}$, $C_{15}$ or $C_{17}$ straight-chain alkyl, with a polypeptide having an average molecular weight of 300 to 2,000.

**9.** Excipient according to Claim 8, characterized in that the dérivatives (IIIb) obtained by using the amides of formula (IVb) wherein $R_7$ stands for a $C_7$ and $C_9$ straight-chain alkyl, represent at most 10% of the sum of the weights of the derivative (IIIa) and the derivatives (IIIb), and that the dérivatives (IIIb) obtained by using the amides of formula (IVb) wherein $R_7$ stands for $C_{13}$, $C_{15}$ and $C_{17}$ straight-chain alkyl represent 17-24%, 6-12% and 5-10% of said sum, respectively.

**10.** Excipient according to Claim 7, 8 or 9, characterized in that the polypeptide is a gelatin hydrolyzate.

**11.** Excipient according to Claims 7, 8, 9 or 10, characterized in that the derivative (IIIa)/ compound (Ia), derivative (IIIa)/compound (Ia) + compound(s) (Ib), derivative (IIIa) + derivative(s) (IIIb)/compound (Ia) and derivative (IIIa) + derivative(s) (IIIb)/compound (Ia) + compound(s) (Ib) weight ratios are each less than or equal to 0.4.

**12.** Excipient according to anyone of the previous Claims, characterized in that it comprises water.

**13.** Excipient according to anyone of the previous Claims, characterized in that it comprises an agent complexing the alkaline-earth ions.

**14.** Excipient according to Claim 13, characterized in that the complexing agent is at least one selected among EDTA, EDTA sodium salts and their mixtures.

**15.** Excipient according to Claim 13 or 14, characterized in that the complexing agent/compound (Ia) and complexing agent/compound (Ia) + compound(s) (Ib) weight ratios are each less than or equal to 0.1.

**16.** Excipient according to anyone of Claims 12 to 15, characterized in that it contains a pH adjusting agent allowing the pH adjustment to 4-7.

**17.** Excipient according to anyone of Claims 12 to 16, characterized in that it comprises :

- compound (Ia) + compounds (Ib) .......... 7.5 - 15 wt%
- compound (IIa) + compounds (IIb) ........ 3 - 6 wt%
- derivative (IIIa) + derivatives (IIIb) .. 0 - 3 wt%
- complexing agent ..................... 0 - 0.5 wt%
- pH adjusting agent ................ to pH 4-7
- deionized water ........................ q.s. 100 wt%

**18.** Antiseptic preparation comprising a water-insoluble organo-chlorinated compound, belonging to the class of carbanilides, and an excipient, characterized in that the excipient is constituted by the one which makes the subject-matter of anyone of Claims 1 to 17.

**19.** Antiseptic preparation according to Claim 18, characterized in that said organo-chlorinated compound is 3,4,4'-trichlorocarbanilide.

**20.** Antiseptic preparation according to Claim 18 or 19, characterized in that it contains 0.5 to 2.5 weight % of said organo-chlorinated compound belonging to the class of carbanilides.

**21.** Antiseptic preparation according to Claim 20, characterized in that it comprises the following components :

- 3,4,4'-trichlorocarbanilide .................. 1.0 wt%
- compound (Ia) + compounds (Ib) ............... 11.5 wt%
- compound (IIa) + compounds (IIb) ............. 4.5 wt%
- derivative (IIIa) + derivatives (IIIb) ....... 0.9 wt%
- complexing agent ........................... 0.3 wt%
- pH adjusting agent ...................... to pH 4-7
- deionized water ........................ q.s. 100 wt%.